Europäisches Patentamt

European Patent Office

Office européen des brevets

(19)

(11) **EP 0 869 360 B1**

(12) **FASCICULE DE BREVET EUROPEEN**

(45) Date de publication et mention
de la délivrance du brevet:
**18.12.2002 Bulletin 2002/51**

(51) Int Cl.⁷: **G01N 33/12**, G01N 27/02

(21) Numéro de dépôt: **98500064.5**

(22) Date de dépôt: **04.03.1998**

(54) **Procédé pour déterminer la composition et la qualité de substances de nature carnée**

Verfahren zur Feststellung von Zusammensetzung und Qualität von Fleischsubstanzen

Method for determining composition and quality of meat material

(84) Etats contractants désignés:
**AT BE DE DK FR IT NL PT**

(30) Priorité: **06.03.1997 ES 9700501**

(43) Date de publication de la demande:
**07.10.1998 Bulletin 1998/41**

(73) Titulaire: **NTE, S.A.**
**08186 Lliça d'Amunt (Barcelona) (ES)**

(72) Inventeurs:
• **Rosell Ferrer, Javier**
**08071 Barcelona (ES)**
• **Pallas, Areny, Ramon**
**08071 Barcelona (ES)**
• **Riu Costa, Pere**
**08071 Barcelona (ES)**
• **Elvira Canas, Jordi**
**08014 Barcelona (ES)**

(74) Mandataire: **Davila Baz, Angel**
**c/o Clarke, Modet & Co.,**
**Goya, 11**
**28001 Madrid (ES)**

(56) Documents cités:
**EP-A- 0 865 763**     **DD-A- 252 443**
**GB-A- 2 272 526**     **GB-A- 2 288 022**
**US-A- 3 966 973**

## Description

La présente invention se réfère à un procédé visant à déterminer la composition et la qualité de substances de nature carnée.

Dans l'industrie de la viande, il est nécessaire d'utiliser des outils objectifs pour déterminer la qualité de la matière première et du produit final résultant. De plus, cet outil doit être fiable et son application doit être faisable dans la chaîne de fabrication. De même, cet outil doit faire en sorte de ne pas altérer la qualité de la substance carnée en raison de la mesure.

Différents systèmes ont été utilisés dans l'industrie de la viande en vue d'obtenir cette classification objective. Dans de nombreux cas, l'industrie dépend d'une série d'experts qui déterminent en dernière instance la qualité de la viande, mais leur verdict est entièrement subjectif. Des mesures de pH 45 minutes après l'abattage de l'animal sont actuellement appliquées pour déterminer certains paramètres de qualité de substances carnées, permettant leur classification. Néanmoins, cette mesure doit être effectuée dans les installations du fournisseur de l'industrie de la viande (abattoirs), ce qui rend donc difficile le suivi de la matière première qui arrive à l'industrie. D'autres mesures de qualité de pièces de viande ont également été faites, mais aucune méthode n'a été capable de fournir une série de paramètres qui donnent une idée de la qualité générale d'une pièce de viande.

De par le document ES 9300378, l'on connaît un procédé pour la détermination de la qualité de substances de nature carnée qui utilise le quotient des modules d'impédance à haute fréquence et à basse fréquence respectivement, ce qui rend indépendante la détermination de la qualité de la viande des facteurs externes, comme par exemple l'impédance de contact des électrodes avec la viande.

Cependant par ce procédé, l'on obtient un résultat dont l'exactitude est assez limitée parce qu'il n'utilise qu'un paramètre, déterminé à partir de deux valeurs expérimentales, pour la décision finale sur la viande.

Le document DD-A-252 443 décrit un procedé pour determiner la composiion te la qualité de substances carnées basé sur la mesure d'une caractéristique dépendente de la capacité des emmbranes biologiques à deux frecuences différentes.

Le document GB-A-2 288 022 décrit un procédé pour déterminer l'état d'une pièce de viande qui est basé sur la mesure pendant un période de temps (ou une schelle de fréquences) des valeurs du voltage (ou de courant) éléctrique transitoire produit par lápplication dún pulse éléctrique.

Le document US-A-3,966,973 décrit un procédé pour determiner la quantité déau des substances carnées qui comprend la mesure de la valeur de l'impédance éléctrique après lápplication dún courant éléctrique d'une fréquence predéterminée.

Le document GB-A-2 272 526 décrit un procédé de termographie qui est basé sur la mesure de l'impédance électrique à plusieurs fréquences différentes.

Par conséquent, la présente invention a pour but d'offrir un procédé qui détermine de façon plus fiable, la composition et en particulier les rapports entre des volumes d'eau intracellulaire et d'eau extracellulaire, ainsi que le rapport entre la masse grasse et la masse maigre d'une pièce carnée sur laquelle l'on effectue la recherche.

Ce travail est résolu grâce au procédé de la présente invention qui permet de mesurer la qualité et la composition de la substance de nature carnée grâce à une série de mesures de l'impédance électrique à plusieurs fréquences, au moins quatre, dans une échelle comprise entre 1 kHz et 10 MHz. Avec les valeurs obtenues, l'on calcule d'abord un paramètre K, donné par le quotient entre le module de l'impédance Z(BF) à une fréquence basse comprise entre 1 kHz et 50 kHz et le module de l'impédance Z(AF) à une fréquence comprise entre 100 kHz et 10 MHz, qui sert d'estimation du rapport entre les volumes d'eau totale et d'eau extracellulaire. Cette valeur est indépendante des conditions d'environnement de la mesure. L'on ajuste ensuite les valeurs expérimentales avec la fonction qui exprime la dépendance de l'impédance électrique (Z) par rapport à la fréquence (f)

$$Z(f) = R_\infty + \frac{R_0 - R_\infty}{1 - \left(j\dfrac{f}{f_c}\right)^{1-\alpha}}$$

par la méthode des minima carrés, dont on détermine les paramètres d'ajustement $R_\infty$, $R_0$, $f_c$ et $\alpha$, qui représentent la résistance à fréquence infinie, la résistance à fréquence zéro, la fréquence à laquelle se produit le maximum de Im(Z) et la dispersion de la taille des cellules du système biologique, respectivement. A partir de ces paramètres d'ajustement, l'on calcule les valeurs suivantes :

a) le rapport entre les volumes d'eau intracellulaire et d'eau extracellulaire, donné par :

$$E(\%) = \frac{R_0 - R_\infty}{R_\infty}$$

b) le contenu de masse maigre MM par l'équation

$$MM = N + M \frac{L_{eq}^2}{Re[(AF)]}$$

N et M étant les coefficients linéaires d'un calibrage préalable pour des viandes présentant des caractéristiques semblables et $L^2_{eq}$ un facteur géométrique effectif donné pour les conditions géométriques de mesure. La masse grasse de la pièce étudiée est obtenue en soustrayant de la masse maigre le poids de la pièce ou échantillon étudié.

L'on explique ci-après en détail la présente invention à l'aide de dessins sur lesquels

la figure 1 montre un spectre du module de l'impédance électrique en fonction de la fréquence f ;

la figure 2 montre un schéma de connexions correspondant à la constellation électrique sur un corps ;

la figure 3 montre deux spectres de l'impédance complexe de deux pièces de nature carnée différentes en fonction de la fréquence :

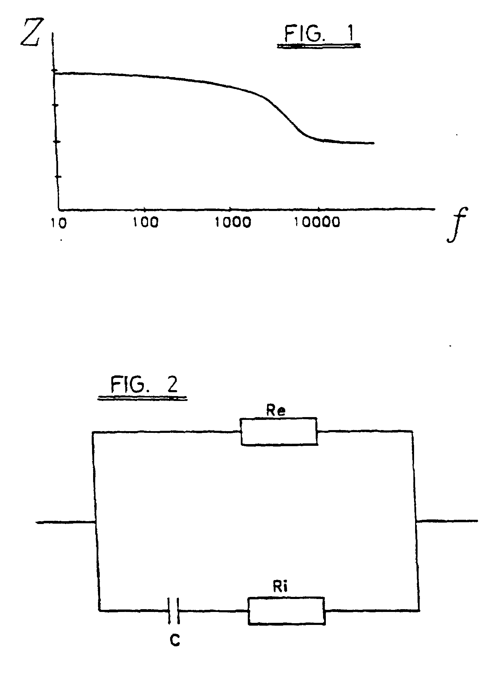

Le procédé faisant l'objet de l'invention est basé sur un phénomène physique dénommé la relaxation au signal d'impédance électrique. Cette relaxation consiste en une chute de la valeur de l'impédance électrique avec la fréquence du signal électrique que l'on utilise pour mesurer l'impédance dans un milieu quelconque. L'impédance électrique d'un milieu se décompose en une partie réelle et une autre partie imaginaire. La partie réelle est connue communément comme la résistance électrique et la partie imaginaire, dans le cas de tissus biologiques, correspond à la capacité électrique du milieu en question. Le phénomène de la relaxation a son explication dans l'absorption d'énergie électromagnétique. L'on accepte par convention un modèle électrique simple où le caractère capacitif de la membrane cellulaire produit cette absorption d'énergie (figure 2). La résistance du milieu extracellulaire est modelée par une résistance Re et la résistance du milieu intracellulaire est modelée par une résistance Ri. La capacité de la membrane cellulaire est modelée par un condensateur C. en mesurant l'impédance électrique sur un circuit comme celui qui est présenté sur la figure 2, l'on obtient un graphique comme celui qui est présenté sur la figure 1. Si le signal que l'on fait passé est à haute fréquence, l'on obtient une impédance électrique égale au parallèle des deux résistances du circuit et si elle est à basse fréquence, elle est égale à la résistance extracellulaire.

La fréquence à laquelle se présente la chute à l'impédance dépend du type de cellule moyenne du système biologique mesuré. Les cellules animales ont une fréquence de relaxation moindre que celle des bactéries. Par conséquent, la mesure de l'impédance électrique à plusieurs fréquences par l'appareil de la présente invention permet de déterminer le type de cellule moyenne existant dans le système biologique.

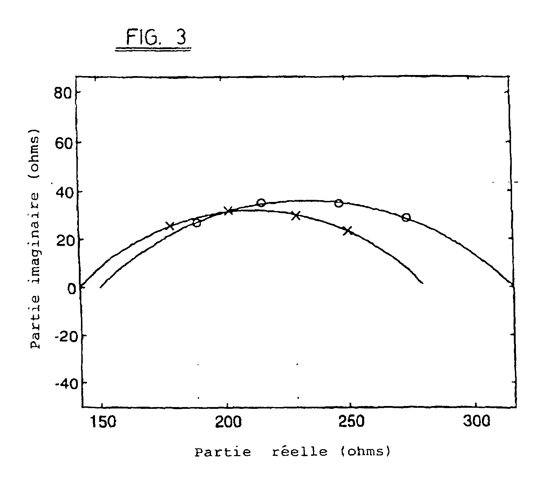

Sur la figure 3, l'on montre une manière de représenter l'impédance d'un système biologique, c'est-à-dire une partie de l'imaginaire de l'impédance en fonction de la partie réelle. Sur cette figure, l'on observe que l'impédance d'un système biologique dessine une demi-circonférence aplatie. L'on peut apprécier la fréquence de coupe comme le point où la partie imaginaire de l'impédance présente la valeur la plus élevée. L'aplatissement de la demi-circonférence est lié à un paramètre appelé $\alpha$. Il y a deux autres paramètres $R_0$ et $R\infty$ qui représentent la valeur de la partie réelle de l'impédance en courant continu et à très haute fréquence (au dessus d'1 MHz). C'est-à-dire, nous disposons de quatre paramètres qui nous décrivent le système biologique mesuré : résistance en courant continu, résistance à fréquence très élevée, fréquence de coupe et indice d'aplatissement de la demi-circonférence.

Par conséquent, le procédé présenté dans cette invention est le suivant :

1) Mesure de l'impédance électrique avec un balayage en fréquence comprise encre 1 kHz et 10 MHz pour obtenir le graphique sur la figure 1. Quatre points de mesure dans l'échelle citée équivalent au nombre minimum de mesures exigé pour reproduire les courbes sur les figures 1 et 3. La mesure se fait en faisant passer un courant éleccrique à fréquence déterminée par deux électrodes connectées à la pièce de viande et en mesurant la chute de tension du courant appliquée au moyen de deux autres électrodes. C'est la base de la mesure d'impédance à 4 fils très résistante aux changements de géométrie. Cette mesure d'impédance se fait avec des courants à plusieurs fréquences jusqu'à obtenir au minimum 4 points de fréquence dans l'échelle encre 1 kHz et 10 MHz.

2) Les mesures d'impédance électrique résultant du balayage en fréquence (au minimum quatre mesures comprises entre 1 kHz et 10 MHz) sont ajustées au moyen d'une courbe

$$Z(f) = R_\infty + \frac{R_0 - R_\infty}{1 - \left(j\frac{f}{f_c}\right)^{1-\alpha}}$$

par un ajustement de minima carrés. Cette équation exprime la dépendance de l'impédance électrique (Z) en fonction de la fréquence (f). Les paramètres $R_0$ et $R_\infty$ sont les résistances que présente le milieu en courant continu et à fréquence très élevée respectivement, $f_c$ est la fréquence à laquelle se produit le maximum de la partie imaginaire et qui dépend du type de cellule moyenne du système biologique ; et $\alpha$ est un paramètre lié à la dispersion de dimensions des cellules du système biologique mesuré. L'expression de ce point 2 reproduit la courbe de la figure 3 que l'on obtiendrait si l'on mesurait une quantité élevée de points.

3) A partir des deux premiers paramètres trouvés ($R_0$ et $R_\infty$), nous obtenons un indice appelé E qui donne le rapport entre eau intracellulaire et eau extracellulaire :

$$E(\%) = \frac{R_0 - R_\infty}{R_\infty} \cdot 100$$

L'impédance mesurée peut être modelée comme le circuit qui est présenté sur la figure 2 décrit précédemment. Les rapports entre les paramètres du circuit (Re et Ri) avec ceux du modèle mathématique sont les suivants :

$$R_e = R_0$$

$$R_i = \frac{R_0 - R_\infty}{R_0 - R_\infty}$$

où $R_0$ et $R_\infty$ sont obtenus à partir du réglage de l'équation où l'on modèle la variation d'impédance à la fréquence y présentée auparavant.

4) Un rapport que l'on peut utiliser au départ également pour produire un indice semblable à E et qui nous donne une idée du rapport entre les volumes d'eau totale et d'eau extracellulaire. L'on obtient le rapport par le quotient de la valeur d'impédance à basse fréquence (entre 1 kHz et 50 kHz) et à haute fréquence (au-dessus de 10 kHz jusqu'à 10 MHz) :

$$K = \frac{|Z\,(BF)|}{|Z\,(AF)|}$$

où $\lceil Z\,(AF)\rfloor$ et $\lceil Z\,(BF)\rfloor$ sont les valeurs du module de l'impédance électrique à haute fréquence et à basse fréquence respectivement.

La température du milieu influe sur la valeur de l'impédance électrique de façon linéaire et calibrée. Par conséquent, il est important de corriger la mesure d'impédance avec la température du système biologique en étude. Comme il s'agit d'un rapport linéaire et calibré, l'on peut facilement corriger l'impédance en trouvant les coefficients linéaires pour le système biologique étudié.

En résumé, i'on obtient une série de paramètres que l'on pourra utiliser pour déterminer la qualité de la viande :

1) rapports E et K qui donnent une idée du rapport entre des volumes d'eaux extracellulaire et intracellulaire.
2) fréquence de coupe, et
3) aplatissement $\alpha$ de la courbe représentée sur la figure 3.

Tout cela devra être calculé en tenant compte de la température de la substance carnée à l'étude. Un autre paramètre important que l'on peut obtenir avec ce procédé est la quantité de tissu maigre d'un échantillon. La quantité de tissu maigre est obtenue à partir de l'équation suivante :

$$MM = Masse\ Maigre = N + M \cdot \frac{L_{eq}^2}{Re[(AF)]}$$

N et M étant des coefficients linéaires d'un calibrage préalable pour des viandes présentant des caractéristiques semblables et $L^2_{eq}$ un facteur géométrique effectif donné par les conditions géométriques de mesure, en obtenant la quantité de masse grasse de la pièce étudiée par soustraction de la quantité de masse maigre du poids de la pièce ou échantillon étudié.

Tous ces paramètres sont utilisés pour déterminer la composition de substances d'origine carnée et par conséquent leur qualité. Tout système biologique carné peut être analysé avec le procédé faisant l'objet de l'invention. Cela signifie que, grâce à ce procédé, un être vivant jusqu'à des pièces de viande peuvent être analysés.

La mesure d'impédance électrique à plusieurs fréquences peut être effectuée au niveau régional. Cela veut dire que l'on peut faire des études locales sur une pièce de viande avec différents muscles. Les paramètres de qualité pourront être donnés par zone analysée de la pièce de viande. Par conséquent, la répartition de fluides et de composés présentant un intérêt comme le sel et la masse maigre peuvent être donnée pour obtenir une carte de composition de la pièce de viande. Préférablement, on procédera suivant la revendication 2.

**Revendications**

1. Procédé servant à déterminer la composition et la qualité de substances de nature carnée en mesurant l'impédance électrique à plusieurs fréquences, au moins quatre, dans l'échelle comprise entre 1 kHz et 1 MHz dans laquelle l'on effectue une première estimation du rapport entre des volumes d'eau totale et d'eau extracellulaire au moyen du quotient K entre le module de l'impédance à basse fréquence Z(BF), située entre 1 kHz et 50 kHz et le module de l'impédance à haute fréquence Z(AF), située entre 100 kHz et 10 MHz, cette valeur étant indépendante des conditions d'environnement de la mesure, procédé **caractérisé par le fait que** dans une première étape l'on effectue un ajustement des points expérimentaux grâce à la méthode de minima carrés par l'expression suivante d'impédance électrique (Z) en fonction de la fréquence (f) du signal électrique :

$$Z(f) = R_\infty + \frac{R_0 - R_\infty}{1 - \left(j\frac{f}{f_c}\right)^{1-\alpha}}$$

en déterminant de la sorte les valeurs $R_\infty$, $R_0$, $f_c$ et $\alpha$, qui représentent la résistance à fréquence infinie, la résistance à fréquence zéro, la fréquence à laquelle se produit le maximum d'Im(Z)et la dispersion des cellules du système biologique, respectivement, avec lesquelles l'on calcule une valeur E associée au rapport entre des volumes d'eau intracellulaire et d'eau extracellulaire, donnée par :

$$E(\%) = \frac{R_0 - R_\infty}{R_\infty} \cdot 100$$

et dans une seconde étape l'on détermine le contenu de masse maigre de la substance de nature carnée sur laquelle l'on doit faire une recherche par une valeur d'impédance obtenue à une fréquence supérieure à 100 kHz, en utilisant l'équation :

$$MM = Masse\ Maigre = N + M \cdot \frac{L_{eq}^2}{Re[(AF)]}$$

N et M étant des coefficients linéaires d'un calibrage préalable pour des viandes présentant des caractéristiques semblables et $L^2_{eq}$ un facteur géométrique effectif donné pour les conditions géométriques de mesure, en obtenant la somme de masse grasse de la pièce étudiée en soustrayant la somme de masse maigre du poids de la pièce ou échantillon étudié.

2. Procédé selon la revendication 1, **caractérisé par le fait que** l'on choisit les zones à étudier de façon à obtenir une carte de caractéristiques qualitatives par régions de la substance carnée sur laquelle l'on fait la recherche.

**Claims**

1. Procedure to determine the composition and quality of meat substances by measuring the electrical impedance at different frequencies, at least four, in the scale ranging from 1 kHz to 1MHz, in which a first estimate is obtained of the ratio between the volumes of total water and extracellular water by the quotient K between the impedance module at low frequency Z(BF), ranging from 1 kHz to 50 kHz and the impedance module at high frequency Z (ZF), ranging from 100 kHz to 10 MHz, where this value is independent of the conditions of the medium measured, the procedure **characterised in that** in a first stage, the experimental points are fitted by a least squares method to the following expression of the electrical impedance (Z) with respect to the frequency (f) of the electrical signal:

$$Z(f) = R\infty + \frac{R_0 - R\infty}{1 - \left(j\dfrac{f}{fc}\right)^{1-\bullet}}$$

determining the values $R\infty$, $R_0$, fc and $\alpha$ that represent respectively the resistance at infinite frequency, the resistance at zero frequency, the frequency at which the maximum value of Im(Z) is obtained and the dispersion of the cells of the biological system, which are used to calculate a value E related to the ratio of volumes of intracellular water and extracellular water, given by:

$$E(\%) = \frac{R_0 - R_\infty}{R_\infty} \times 100$$

and, in a second stage, the lean matter contents of the meat substance being studied is determined by finding the impedance value obtained at a frequency above 100 kHz, according to the equation:

$$LM = Lean\ matter = N + M\ \ \frac{L_{eq}^2}{Re[(AF)]}$$

where N and M are the linear coefficients of a previous calibration for meat with similar characteristics and $L^2_{eq}$ is an effective geometric factor for geometric measuring conditions, obtaining the fat contents of the cut of meat studied by subtracting the lean matter from the weight of the cut of meat or sample being studied.

2. Procedure according to Claim 1, **characterised in that** the zones to be studied are selected in order to obtain a record of the qualitative characteristics for each zone of the meat under study.

**Patentansprüche**

1. Verfahren zur Bestimmung der Zusammensetzung und der Qualität von fleischartigen Substanzen durch Messen der elektrischen Impedanz bei wenigstens vier Frequenzen im Bereich zwischen 1 kHz und 1 MHz, bei dem eine erste Abschätzung des Verhältnisses zwischen dem Volumen des gesamten Wassers und des extrazellulären Wassers durchgeführt wird mit Hilfe des Quotienten K des Impedanzmoduls bei tiefer Frequenz Z(BF), die zwischen 1 kHz und 50 kHz beträgt, und dem Impedanzmodul bei hoher Frequenz Z(AF), die zwischen 100 kHz und 10 MHz beträgt, wobei dieser Wert unabhängig von den Umgebungsbedingungen der Messung ist, **gekennzeichnet durch** die Tatsache, dass bei einem ersten Verfahrensschritt mit der Methode der kleinsten Quadrate mittels des folgenden Ausdrucks der elektrischen Impedanz (Z) als Funktion der Frequenz (f) des elektrischen Signals eine Anpassung der Experimentierpunkte vorgenommen wird:

$$Z(f) = R_\infty + \frac{R_0 - R_\infty}{1 - \left(j\frac{f}{f_c}\right)^{1-\alpha}}$$

wobei auf diese Weise die Werte $R_\infty$, $R_0$, $f_c$ und $\alpha$ bestimmt werden, die den Widerstand bei unendlicher Frequenz, den Widerstand bei Frequenz Null, die Frequenz, bei der das Maximum von Im(Z) gegeben ist, bzw. die Verteilung der Zellen des biologischen Systems darstellen, mit denen ein Wert E berechnet wird, der mit dem Verhältnis zwischen den Volumina des intrazellulären Wassers und des extrazellulären Wassers zusammenhängt und der gegeben ist **durch**:

$$E(\%) = \frac{R_0 - R_\infty}{R_\infty} \cdot 100$$

und **durch** einen zweiten Verfahrensschritt, in dem der Gehalt an fettfreier Masse der fleischartigen Substanz, die untersucht werden soll, bestimmt wird **durch** einen Impedanzwert, der bei einer Frequenz oberhalb von 100 kHz unter Verwendung der Gleichung:

$$MM = \text{fettfreie Masse} = N - M \cdot \frac{L_{eq}^2}{\text{Re}[(AF)]}$$

erhalten wird, wobei N und M lineare Koeffizienten einer vorläufigen Eichung für Fleisch, das vergleichbare Charakteristika aufweist, sind und wobei $L_{eq}^2$ ein für die geometrischen Bedingungen der Messung gegebener effektiver Geometriefaktor ist, wobei die Summe der fetthaltigen Masse des untersuchten Stücks **durch** Subtraktion der Summe der fettfreien Masse von dem Gewicht des untersuchten Stücks oder der untersuchten Probe erhalten wird.

2. Verfahren nach Anspruch 1, **gekennzeichnet durch** die Tatsache, dass die zu untersuchenden Bereiche so gewählt werden, dass eine Karte mit qualitativen Charakteristika nach Bereichen der untersuchten fleischartigen Substanz erhalten wird.

$Z$

FIG. 1

10    100    1000    10000    $f$

FIG. 2

Re

Ri

C

FIG. 3

Partie imaginaire (ohms)

Partie réelle (ohms)